# EUROPÄISCHE PATENTSCHRIFT

(11) **EP 0 795 339 B1**
(45) Veröffentlichungstag und Bekanntmachung des Hinweises auf die Patenterteilung: **06.11.2002**
(21) Anmeldenummer: 97104214.8
(22) Anmeldetag: 13.03.1997
(51) Int. Cl.: A61M 25/00

(54) **Katheter zur Harnableitung**
Urine drainage catheter
Cathéter pour l'évacuation d'urine

(30) Priorität: 13.03.1996 DE 19609714
(43) Veröffentlichungstag der Anmeldung: 17.09.1997
(73) Patentinhaber: VIA LOG Medikalprodukte GmbH Kosmetik - Medien, D-75378 Bad Liebenzell (DE)
(72) Erfinder: Hutzler, Martin, 75378 Bad Liebenzell (DE)
(74) Vertreter: KOHLER SCHMID + PARTNER

(56) Entgegenhaltungen:
- EP-A- 0 384 476
- DE-A- 3 441 586
- DE-C- 330 284

## Beschreibung

Die Erfindung geht aus von einem Katheter zur Harnableitung mit einem flexiblen Katheterschaft, der patientenseitig in einer sich konisch verjüngenden Katheterspitze endet.

Ein derartiger Katheter ist durch die EP 0 384 476 B1 bekanntgeworden.

Zum Katheterismus der Harnblase werden Gummi- oder Kunststoffkatheter verwendet. Sie dienen in erster Linie der Harnableitung. Die röhrenförmigen Instrumente sind einläufig und weisen in Spitzennähe Augen für die Harnableitung und endständig einen Trichter als Verbindungselement an abführende Geräte bzw. zu Auffangbehältnissen auf. Benötigt werden die bekannten Katheter von Personen, bei denen eine Blasenfunktionsstörung vorliegt. Jeder Katheterismus muß unter strenger Beachtung der Regeln der Asepsis durchgeführt werden, hat schonend und vorsichtig zu erfolgend und wird vom Arzt oder Pflegepersonal oder von dem Patienten durch Selbstkatheterisierung durchgeführt.

Der bekannte Katheter zur Harnableitung ist mit einer Katheterspitze ausgebildet, die in einer kugelförmigen Spitze endet, die über einen halsförmigen Abschnitt an einen sich konisch verjüngenden Abschnitt der Katheterspitze angeformt ist. Die Katheterspitze selbst weist höckerförmige Erhebungen auf, wobei die Höcker über den Umfang der Katheterspitze verteilt angeordnet sind. In den Räumen zwischen den Höckern kann sich Gleitmittel in einem begrenzten Umfang ansammeln, das beim Katherismus zur Schonung der Urethralschleimhaut und der Mm. sphincter vesicae beiträgt.

Der Erfindung liegt die Aufgabe zugrunde, einen Katheter zur Harnableitung derart weiterzubilden, daß beim Einführen des Katheters in die Harnröhre oder das Urostoma die den Katheter umgebende Schleimhaut und die Mm. sphincter vesicae noch ausgeprägter geschont werden.

Diese Aufgabe wird erfindungsgemäß dadurch gelöst, daß am Umfang der Katheterspitze über den Umfang der Katheterspitze verteilt, sich in axialer Richtung erstreckende, rinnenförmige Ausnehmungen ausgebildet sind.

Der erfindungsgemäße Katheter zur Harnableitung hat mit einer derart ausgebildeten Katheterspitze den Vorteil, daß die Ausnehmungen ein Gleitmitteldepot bilden können, das einerseits beim Katheterismus so viel Gleitmittel zur Verfügung stellen kann, daß die Reibungswerte zwischen der Urethralschleimhaut und der Mm. sphincter vesicae bzw. dem Urostoma immer möglichst klein gehalten werden können und daß andererseits die Katheterspitze derart ausgebildet ist, daß sie aufgrund ihrer Formgebung keine Irritationen beim Katheterismus erzeugt. Von der erfindungsgemäßen Spitze kann auch in die Harnröhre bzw. das Urostoma injiziertes Gleitmittel aufgenommen und danach wieder sukzessive abgegeben werden. Damit unterstützt die erfindungsgemäße Katheterspitze die gleichmäßige Verteilung des Gleitmittels während eines Katheterismus über den gesamten Verlauf der Harnröhre bzw. des Urostomas.

Die Ausnehmungen sind bevorzugt als Vertiefungen ausgebildet, die in axialer Erstreckung nahezu die gesamte Länge der Katheterspitze abdecken. Die jeweilige Ausnehmung weist dabei eine Tiefe auf, die zum Katheterschaft hin zunimmt. Dies hat den Vorteil, daß beim Einführvorgang des Katheters in die Harnröhre bzw. Urostoma kontinuierlich Gleitmittel aus dem Depot abgegeben werden kann. Das kontinuierlich abgegebene Gleitmittel verteilt sich auf der der Katheterspitze in Bewegungsrichtung nachfolgenden Oberfläche des Katheters gleichmäßig und gewährleistet somit einen sanften Katheterismus.

Bei einer bevorzugten Weiterbildung der Erfindung sind an der Katheterspitze mindestens zwei Ausnehmungen, gleichmäßig über den Umfang der Katheterspitze verteilt, ausgebildet.

Dies hat den Vorteil, daß über derart angeordnete Ausnehmungen es stets gewährleistet ist, daß die Außenoberfläche der Katheterspitze und des in Bewegungsrichtung nachfolgenden Katheterschaftes mit Gleitmittel benetzt ist bzw. bei einem Bewegungsvorschub in der Harnröhre stets Gleitmittel flächendeckend abgegeben wird.

In einer Weiterbildung der Erfindung sind zwischen den Ausnehmungen Flächenabschnitte vorgesehen, deren Oberfläche zahlreiche zur Außenoberfläche hin abgerundete Einbuchtungen aufweisen, die ein auf der Katheterspitze verteiltes Gleitmittel in Filmschichtdicke aufnehmen können.

Dies hat den Vorteil, daß das von den Ausnehmungen abgegebene Gleitmittel auch ausreichend stark auf ausnehmungsfreien Flächen der Katheterspitze haften kann, so daß zwischen diesen Flächenabschnitten und der angrenzenden Urethralschleimhaut keine großflächigen Adhärenzen auftreten können. Diese Ausgestaltung der Flächenabschnitte begünstigt einen schonenden Katheterismus zusätzlich. Die nach außen abgerundeten Einbuchtungen gleiten in der Harnröhre schleimhautschonend und verursachen keine zusätzlichen Irritationen.

In weiterer Ausgestaltung der Erfindung geht die Katheterspitze ansatzlos in den Katheterschaft über.

Dies hat den Vorteil, daß obwohl die Katheterspitze und der Katheterschaft nicht einstückig aus einem Material ausgeformt sind, es beim Katheterismus zu keiner zusätzlichen Belastung der Schleimhäute kommt. Vielmehr kann durch eine unterschiedliche Materialkombination von Katheterschaft und Katheterspitze auf unterschiedlichste Bedürfnisse beim Katheterismus eingegangen werden. Der Katheterspitze kann ein zum Katheterschaft unterschiedliches Flexibilitätsverhalten zugeordnet werden.

In weiterer Ausgestaltung der Erfindung ist in der Katheterspitze ein Hohlraum ausgebildet, der Durchgangsöffnungen zu den Ausnehmungen aufweist.

Dies hat den Vorteil, daß in der Katheterspitze ein Gleitmitteldepot angelegt werden kann, das bei entsprechender Aktivierung das Gleitmittel in die Ausnehmungen abgibt. Denkbar sind verschiedenste Ausführungsformen. So kann beispielsweise die Katheterspitze über eine Hülse verschlossen sein. Wird die Hülse von der Katheterspitze abgezogen, so kann das im Hohlraum unter Druck gespeicherte Gleitmittel in die Ausnehmungen ausströmen, und eine Benetzung der Katheterspitze mit Gleitmittel von außen ist nicht mehr notwendig. Diese Ausgestaltung einer Katheterspitze vereinfacht die gesamte Handhabung des Katheters beim Katheterismus zusätzlich.

In weiterer Ausgestaltung der Erfindung sind die Ausnehmungen mit abgerundeten Ränder ausgebildet.

Dies hat den Vorteil, daß die Ausnehmungen selbst keine zusätzliche Belastung beim Katheterismus darstellen. Weiterhin ist es vorteilhaft, wenn die Ausnehmungen untereinander verbunden sind. Durch eine derartige Ausgestaltung kann Gleitmittel von einer Ausnehmung in die andere Ausnehmung unmittelbar strömen und Gleitmittelmengendefizite in einer Ausnehmung können über einen Zufluß aus einer anderen Ausnehmung ausgeglichen werden.

Es versteht sich, daß die vorstehend genannten und die nachstehend noch erläuterten Merkmale nicht nur in der angegebenen Kombination, sondern auch in anderen Kombinationen oder in Alleinstellung einsetzbar sind, ohne den Rahmen der vorliegenden Erfindung zu verlassen.

Weitere Vorteile ergeben sich aus der Beschreibung und der beigefügten Zeichnung.

Ausführungsbeispiele der Erfindung sind in den nachfolgenden Figuren der Zeichnung dargestellt. Es zeigt:
- Fig. 1: eine perspektivische Darstellung einer erfindungsgemäßen Katheterspitze eines Katheters zur Harnableitung;
- Fig. 2: einen Längsschnitt durch eine erfindungsgemäße Katheterspitze eines Katheters zur Harnableitung;
- Fig. 3: eine Ansicht gemäß III-III der Fig. 2;
- Fig. 4: eine Ansicht gemäß eines Schnitts IV-IV der Fig. 2;
- Fig. 5: einen Schnitt gemäß V-V der Fig. 2;
- Fig. 6: einen Schnitt durch eine weitere Ausführungsform einer erfindungsgemäßen Katheterspitze mit weiteren Ausnehmungen und einem Hohlraum.

Die einzelnen Figuren der Zeichnung zeigen den erfindungsgemäßen Gegenstand nicht maßstäblich. Die gegenständlichen Ausgestaltungen in den einzelnen Figuren sind teilweise stark vergrößert, schematisiert und vereinfacht dargestellt.

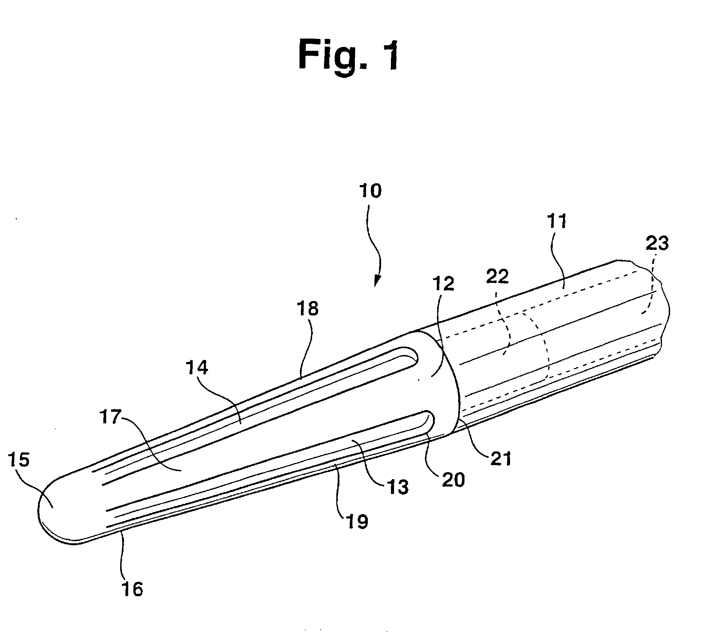

In Fig. 1 ist mit 10 ein Katheter zur Harnableitung gezeigt, bei dem ein Katheterschaft 11 nur teilweise dargestellt ist. Der Katheterschaft 11 weist in bekannter Weise Augen und am patientenabgewandten Ende einen Trichter auf, der mit einem geeigneten Auffangbehältnis verbunden werden kann. Der Katheterschaft 11 ist am patientenseitigen Ende mit einer Katheterspitze 12 versehen, die in den Katheterschaft 11 eingefügt ist.

Die Katheterspitze 12 weist an ihrer Außenoberfläche Ausnehmungen 13, 14 auf, die an der Katheterspitze 12 axial erstreckend ausgebildet sind und rinnenförmig verlaufen. Die Ausnehmungen 13, 14 sind als Vertiefungen in der Katheterspitze 12 ausgebildet.

Die Katheterspitze 12 endet in einer kugelförmigen Verdikkung 15, die über einen kurzen, halsförmigen Abschnitt 16 an einem sich konisch verjüngenden Abschnitt der Katheterspitze 12 angeformt ist. Die kugelförmige Verdickung 15 ist damit hoch elastisch an der konischen Verjüngung der Katheterspitze 12 angeformt, ohne daß dadurch die Richtungsstabilität der erfindungsgemäßen Katheterspitze 12 eingeschränkt ist.

Zwischen den Ausnehmungen 13, 14 und denen in der Fig. 1 nicht sichtbaren Ausnehmungen auf der Rückseite der Katheterspitze 12 sind Flächenabschnitte 17, 18, 19 ausgebildet, die die Ausnehmungen 13, 14 bzw. die nicht sichtbaren Ausnehmungen miteinander verbinden. Diese Flächenabschnitt 17, 18, 19 weisen unter vergrößerter Betrachtung eine hügelige Oberflächenstruktur auf, die in ihren Tälern Gleitmittel in Filmschichtdicke speichern kann.

Die Ausnehmungen 13, 14 bzw. die nicht sichtbaren Ausnehmungen weisen gegenüber den angrenzenden Flächenabschnitten 17, 18, 19 Ränder 20 auf, die abgerundet sind.

Die Katheterspitze 12 ist formschlüssig und möglicherweise auch materialschlüssig mit dem Katheterschaft 11 verbunden. Ein Übergang 21 ist linienförmig aber stufenfrei erkennbar. D.h., die Katheterspitze 12 geht ansatzlos in den Katheterschaft 11 über. In der Figur ist noch ein Zapfen 22 der Katheterspitze 12 gezeigt (mit gestrichelten Linien), der in ein Lumen 23 des Katheterschaftes 11 ragt. Über den Zapfen 22 kann eine Katheterspitze 12 in den Katheterschaft 11 form- und kraftschlüssig eingefügt werden.

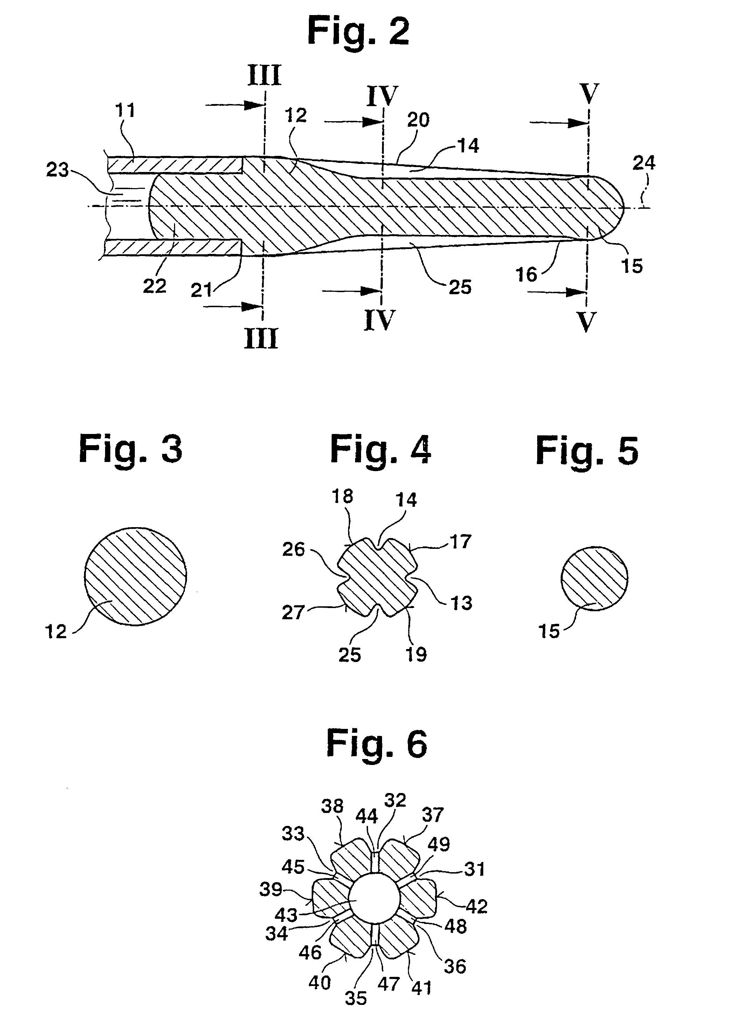

Fig. 2 zeigt einen Längsschnitt durch eine erfindungsgemäße Katheterspitze 12. Über den Zapfen 22 ist die Katheterspitze 12 in den Katheterschaft 11 eingefügt. Der Katheterschaft 11 ist nur in einer kurzen axialen Erstreckung gezeigt, und das Lumen 23 des Katheterschaftes 11 ist in der Figur 2 ebenfalls nur ansatzweise erkennbar. Die Katheterspitze 11 ist stufenfrei am Übergang 21 in den Katheterschaft 11 eingefügt. Die Katheterspitze 12 verjüngt sich konisch zur kugelförmigen Verdickung 15 hin. Die Katheterspitze 12 und der Katheterschaft 11 weisen eine gemeinsame Achse 24 auf, die geradlinig verläuft. Die in der Figur sichtbaren Ausnehmungen 14, 25 sind als Vertiefungen ausgebildeten, deren Volumina sich zum Katheterschaft 11 hin vergrößern. Die Ausnehmungen 14, 25 sind durch abgerundete Ränder 20 begrenzt. Die kugelförmige Verdickung 15 ist über einen halsförmigen Abschnitt 16 an die konische Verjüngung der Katheterspitze 12 angeformt. Über diese Maßnahme läßt sich die kugelförmige Verdickung 15 aus ihrer vorgegebenen Lage richtungsführend auslenken, sofern radial gerichtete Kräfte auf die kugelförmige Verdickung 15 wirken.

Figur 3 zeigt eine Ansicht gemäß III-III der Fig. 2. Diese Ansicht zeigt, daß die Katheterspitze 12 aus einem Vollmaterial besteht und daß im Übergangsbereich der Katheterspitze 12 zum Katheterschaft 11 keine Ausnehmungen ausgebildet sind. Die Oberfläche ist in diesem Bereich der Katheterspitze 12 derart strukturiert, daß ein auf dieser Oberfläche aufgebrachtes Gleitmittel filmartig haftet und daß dadurch die Gleiteigenschaften in der Harnröhre bzw. dem Urostoma verbessert werden.

Fig. 4 zeigt eine Ansicht gemäß dem Schnitt IV-IV der Fig. 2. Die Katheterspitze 12 ist aus einem körperverträglichen elastischen Kunststoffmaterial hergestellt, und dieser Schnitt zeigt, daß bei dieser Ausführungsform Ausnehmungen 13, 14, 25, 26 ausgebildet sind. Diese Ausnehmungen 13, 14, 25, 26 sind über Flächenabschnitte 17, 18, 19, 27 voneinander beabstandet. Die Flächenabschnitte 17, 18, 19, 27 sind mit Einbuchtungen auf der Oberfläche versehen, die Gleitmittel in Filmschichtstärke speichern können.

Fig. 5 zeigt einen Schnitt gemäß V-V der Fig. 2. Dieser Schnitt zeigt, daß in diesem Bereich die an der Katherspitze 12 ausgebildeten Ausnehmungen 14, 25 auslaufend enden. Die kugelförmige Verdickung 15 weist ebenfalls eine Oberflächenstruktur auf, die Gleitmittel in Filmschichtdicke haftend aufnehmen kann.

Fig. 6 zeigt eine weitere Ausführungsform einer Katheterspitze 30 in einem Schnitt quer zur axialen Erstreckung der Katheterspitze 30, die verschiedene Ausnehmungen 31, 32, 33, 34, 35, 36 erkennen läßt. Die Ausnehmungen 31-36 sind über Flächenabschnitte 37, 38, 39, 40, 41, 42 voneinander beabstandet. In den Ausnehmungen 31-36 läßt sich ein Gleitmitteldepot anlegen, das sukzessive Gleitmittel in einem stets ausreichenden Umfang an die Oberfläche der Katheterspitze 30 bzw. an die der Katheterspitze 30 nachfolgenden Oberflächen abgeben kann. Die Katheterspitze 30 weist zusätzlich gegenüber der schon beschriebenen Katheterspitze einen Hohlraum 43 auf, der ein Gleitmitteldepot für alle an der Katheterspitze 30 ausgebildeten Ausnehmungen 31-36 bildet. Der Hohlraum 43 ist über Durchgangsöffnungen 44-49 mit den Ausnehmungen 31-36 verbunden. Ein in dem Hohlraum 43 gespeichertes Gleitmittel kann bei entsprechender Aktivierung des Gleitmitteldepots über die Durchgangsöffnungen 44-49 an die Ausnehmungen 31-36 abgegeben werden.

Ein Katheter (10) zur Harnableitung weist einen flexiblen Katheterschaft (11) auf, der patientenseitig in einer sich konisch verjüngenden Katheterspitze (12) endet. Die Katheterspitze (12) ist am Außenumfang mit in axialer Richtung erstreckenden rinnenförmigen Ausnehmungen (13, 14) versehen, die ein Gleitmitteldepot darstellen. In den Ausnehmungen (13, 14) kann Gleitmittel für einen Katheterismus gespeichert werden. Das Gleitmittel wird bei einer axialen Verschiebung der Katheterspitze (12) in der Harnröhre an die umgebende Urethralschleimhaut bzw. an die Oberflächen der Katheterspitze bzw. des Katheterschaftes (11) angegeben.

## Patentansprüche

1. Katheter zur Harnableitung mit einem flexiblen Katheterschaft (11), der patientenseitig in einer sich konisch verjüngenden Katheterspitze (12; 30) endet, **dadurch gekennzeichnet, daß** am Umfang der Katheterspitze (12; 30) über den Umfang der Katheterspitze (12; 30) verteilt sich in axialer Richtung erstreckende, rinnenförmige Ausnehmungen (13, 14; 25; 26; 31, 32, 33, 34, 35, 36) ausgebildet sind.

2. Katheter nach Anspruch 1, **dadurch gekennzeichnet, daß** die jeweilige Ausnehmung (13, 14; 25; 26; 31-36) eine Tiefe aufweist, die zum Katheterschaft (11) hin zunimmt.

3. Katheter nach Anspruch 1 oder 2, **dadurch gekennzeichnet, daß** an der Katheterspitze (12; 30) mindestens zwei Ausnehmungen (13, 14; 25; 26; 31-36), gleichmäßig über den Umfang der Katheterspitze (12; 30) verteilt, ausgebildet sind.

4. Katheter nach einem der Ansprüche 1 bis 3, **dadurch gekennzeichnet, daß** zwischen den Ausnehmungen (13, 14; 25; 26; 31-36) Flächenabschnitte (17, 18, 19; 27; 37, 38, 39, 40, 41, 42) vorgesehen sind, deren Oberfläche zahlreiche zur Außenoberfläche hin abgerundete Einbuchtungen aufweisen, die ein auf der Katheterspitze (12; 30) verteiltes Gleitmittel in Filmschichtdicke aufnehmen können.

5. Katheter nach einem der Ansprüche 1 bis 4, **dadurch gekennzeichnet, daß** die Katheterspitze (12; 30) in den Katheterschaft (11) ansatzlos übergeht.

6. Katheter nach einem der Ansprüche 1 bis 5, **dadurch gekennzeichnet, daß** in der Katheterspitze (12; 30) ein Hohlraum (43) ausgebildet ist, der Durchgangsöffnungen (44, 45, 46, 47, 48, 49) zu den Ausnehmungen (31, 32, 33, 34, 35, 36) aufweist.

7. Katheter nach einem der Ansprüche 1 bis 6, **dadurch gekennzeichnet, daß** die Ausnehmungen (13, 14; 25; 26; 31-36) Ränder (20) aufweisen, die abgerundet sind.

## Claims

1. A catheter for draining off urine, with a flexible catheter stem (11) which, at the patient end, terminates in a conically tapering catheter tip (12; 30), **characterised in that** extending in an axial direction and distributed around the periphery of the catheter tip (12; 30) there are channel-like recesses (13, 14; 25; 26; 31, 32, 33, 34, 35, 36).

2. A catheter according to Claim 1, **characterised in that** the relevant recess (13, 14; 25, 26; 31-36) has a depth which increases in the direction of the catheter stem (11).

3. A catheter according to Claim 1 or 2, **characterised in that** at the catheter tip (12; 30) there are at least two recesses (13, 14; 25; 26; 31-36) evenly distributed over the periphery of the catheter tip (12; 30).

4. A catheter according to one of Claims 1 to 3, **characterised in that** between the recesses (13, 14; 25; 26; 31-36) there are surface portions (17, 18, 19; 27; 37, 38, 39, 40, 41, 42) the surface of which has numerous indentations which are rounded off towards the outer surface and which are capable of holding a film-thickness layer of lubricant distributed over the catheter tip (12; 30).

5. A catheter according to one of Claims 1 to 4, **characterised in that** the catheter tip (12; 30) merges steplessly into the catheter stem (11).

6. A catheter according to one of Claims 1 to 5, **characterised in that** there is in the catheter tip (12; 30) a cavity (43) comprising apertures (44, 45, 46, 47, 48, 49) extending through to the recesses (31, 32, 33, 34, 35, 36).

7. A catheter according to one of Claims 1 to 6, **characterised in that** the recesses (13, 14; 25; 26; 31-36) have edges (20) which are rounded off.

## Revendications

1. Cathéter pour l'évacuation d'urine comportant une tige de cathéter flexible (11) qui se termine du côté du patient par une pointe de cathéter (12 ; 30) à rétrécissement conique, **caractérisé en ce que** des évidements en forme de rigoles (13, 14 ; 25 ; 26 ; 31, 32, 33, 34, 35, 36) qui s'étendent en direction axiale sont formés sur la périphérie de la pointe de cathéter (12 ; 30) en étant répartis sur la périphérie de la pointe de cathéter (12 ; 30).

2. Cathéter selon la revendication 1, **caractérisé en ce que** chaque évidement (13, 14 ; 25 ; 26 ; 31-36) présente une profondeur qui augmente en direction de la tige de cathéter (11).

3. Cathéter selon la revendication 1 ou 2, **caractérisé en ce qu'**au moins deux évidements (13, 14 ; 25 ; 26 ; 31-36), qui sont répartis régulièrement sur la périphérie de la pointe de cathéter (12 ; 30), sont formés sur la pointe de cathéter (12 ; 30).

4. Cathéter selon l'une des revendications 1 à 3, **caractérisé en ce qu'**il est prévu entre les évidements (13, 14 ; 25 ; 26 ; 31-36) des sections de surface (17, 18, 19 ; 27 ; 37, 38, 39, 40, 41, 42) dont la surface comporte de nombreuses échancrures arrondies en direction de la surface externe, qui peuvent recevoir en une épaisseur de couche de film un lubrifiant réparti sur la pointe de cathéter (12 ; 30).

5. Cathéter selon l'une des revendications 1 à 4, **caractérisé en ce que** la pointe de cathéter (12 ; 30) se transforme en la tige de cathéter (11) sans saillie.

6. Cathéter selon l'une des revendications 1 à 5, **caractérisé en ce qu'**une cavité (43) qui comporte des ouvertures de passage (44, 45, 46, 47, 48, 49) vers les évidements (31, 32, 33, 34, 35, 36) est formée dans la pointe de cathéter (12 ; 30).

7. Cathéter selon l'une des revendications 1 à 6, **caractérisé en ce que** les évidements (13, 14 ; 25 ; 26 ; 31-36) comportent des bords (20) qui sont arrondis.
